Europäisches Patentamt

European Patent Office

Office européen des brevets

(19)

(11) **EP 1 584 243 A1**

(12) **EUROPEAN PATENT APPLICATION**
published in accordance with Art. 158(3) EPC

(43) Date of publication:
**12.10.2005 Bulletin 2005/41**

(21) Application number: **03774135.2**

(22) Date of filing: **21.11.2003**

(51) Int Cl.⁷: **A23K 1/16**, A23K 1/17,
C12N 9/42, C12N 9/26,
C12N 9/50, C12N 9/20,
C12N 9/96, C12N 9/98

(86) International application number:
**PCT/JP2003/014895**

(87) International publication number:
**WO 2004/047550 (10.06.2004 Gazette 2004/24)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **22.11.2002 JP 2002338864**

(71) Applicant: **Meiji Seika Kaisha, Ltd.
Tokyo 104-8002 (JP)**

(72) Inventors:
• **KOYAMA, Toshikazu,
Pharmaceutical Research Center
Yokohama-shi, Kanagawa 222-8567 (JP)**
• **NAKAMURA, Satoshi,
Pharmaceutical Research Center
Yokohama-shi, Kanagawa 222-8567 (JP)**

• **MIWA, Takehiro,
Pharmaceutical Research Center
Yokohama-shi, Kanagawa 222-8567 (JP)**
• **KIMURA, Fumio,
Pharmaceutical Research Center
Yokohama-shi, Kanagawa 222-8567 (JP)**
• **KIKUCHI, Hiroshi
Iwaki-shi, Fukushima 970-8043 (JP)**
• **KUROSAKI, Kazuo, Ohira Co-op 103, 38-17
Iwaki-shi, Fukushima 971-8182 (JP)**

(74) Representative:
**Gille Hrabal Struck Neidlein Prop Roos
Patentanwälte
Brucknerstrasse 20
40593 Düsseldorf (DE)**

(54) **GRANULAR COMPOSITION AND PROCESS FOR PRODUCING THE SAME**

(57) The present invention intends to provide a granular composition which is stable at high temperature and high humidity and to provide a simple method of efficiently producing such a granular composition. According to the present invention, there is provided a granular composition containing a core material and a layer that covers the core material, in which the core material is made of saccharides and the layer that covers the core material contains a bioactive ingredient and a hardened oil. In addition, there is provided a method of producing the granular composition, including allowing a mixture of a molten hardened oil and a bioactive ingredient to adhere to a granular saccharide or to be formed into a film thereon.

**EP 1 584 243 A1**

**Description**

Technical Field

The present invention relates to a granular composition and a method of producing the same. More specifically, the present invention relates to a granular composition containing a bioactive ingredient with enhanced wet-heat stability and to a method of producing such a granular composition such that a mixture of a molten hardened oil and a bioactive ingredient is adhered to granular saccharides or processed into a film thereon.

Background Art

Bioactive ingredients, which include enzymes, antibiotics, vaccines, hormones, and vitamins, are generally in the form of powders in the dry state. Under such a condition, the bioactive ingredients are used in pharmaceutical preparations, foods, detergents, feed for domestic animals, and so on. However, the bioactive ingredients are practically unfavorable because of their poor fluidity and dusting characteristics which may cause allergy symptoms or the like through inhalation, skin-contact, or the like. Therefore, if necessary, those ingredients may be used after being processed in predetermined forms.

In addition, when the bioactive ingredients are added to feed for domestic animals, the activities of the bioactive ingredients generally decrease at high temperature and high humidity. Furthermore, in feed mills, toxic bacteria such as Salmonella which are commonly found in feed and raw materials thereof at feed-producing factory are sterilized. Therefore, for pelletizing the feed, the feed is subjected to a thermal treatment with steam at about 80°C. On the other hand, in summer, an outside feed-reserving tank may reach at a temperature in excess of 50°C and a humidity in excess of 80%.

From the above facts, persons having ordinary skill in the art have demanded stable bioactive ingredient-containing pharmaceutical preparations from which powdery bioactive ingredients are hardly scattered and whose activity does not show a drastic decrease at high temperature and high humidity. Therefore, for example, granular enzymatic compositions are the forms which have been used in the art comparatively frequently because of ease in handling. The granular enzymatic compositions have been produced by means of extrusion granulation, roll granulation, and the like.

For instance, there is reported a method of producing a granular or fine granular enzyme, where a mixture of an enzyme and a particulate substance having a mesh particle size of 20-100 (particle size of 840-150 μm) and low melting point is heated up to the melting point or more of the substance having low melting point under fluidized condition to granulate the mixture (JP 04-13019 B).

In addition, as a method of preventing an enzyme from scattering, there are several methods for making the enzyme into a granular pharmaceutical preparation, such as one in which granules are produced using cellulose as a core material (JP 01-112983 A), one in which granules are produced using a substance having low melting point as a core material (JP 58-214333 A), one in which a solution dissolving a binder material and an enzyme is sprayed on a core (JP 60-37983 A and JP 60-37984 A), one in which a core material composed of saline and/or saccharides is covered with both a substance having low melting point and an enzyme, which are being dissolved, and then added with a substance having high melting point (JP 03-64108 B). However, any of those methods has disadvantages in that a production process is complicated and the stability of an enzyme at high temperature and high humidity is low.

Furthermore, as a method of retaining the stability of an enzyme at high temperature and high humidity, there is provided a method involving coating a core material containing an enzyme with both a hydrophobic substance and a water-insoluble substance (JP 11-514240 A). However, this method requires a complicated production process and takes a long time in production, while a granulating machine is limited to a particular type.

The present invention intends to provide a granular composition which is stable at high temperature and high humidity with respect to any one of bioactive ingredients such as antibiotics, vaccines, hormones and vitamins besides enzymes, and to provide a simple method of efficiently producing such a granular composition.

Disclosure of the Invention

Bioactive ingredients, as effective ingredients of granular compositions, generally used are those granulated by a spray drying process and so on. On the other hand, the inventors of the present invention have found that a structure in which load of wet heat does not directly affect the bioactive ingredient can be obtained by: utilizing the fact that fats have the properties of being molten and solidified at predetermined temperatures; and suspending bulk powder of such a bioactive ingredient in fats to allow the bulk powder to adhere to an appropriate carrier or to be formed into a film thereon. Furthermore, the inventors of the present invention have completed the present invention by developing a method of simply producing such a granular composition with a reduced number of steps.

The present invention according to claim 1 provides a granular composition including a core material and a layer that covers the core material, in which the core material is made of saccharides and the layer that covers the core material is made of a hardened oil and a bioactive ingredient.

The present invention according to claim 2 provides the granular composition according to claim 1, in which the saccharide is granulated sugar or lactose.

The present invention according to claim 3 provides the granular composition according to claim 1, in which the hardened oil is a hardened palm oil.

The present invention according to claim 4 provides the granular composition according to claim 1, in which the bioactive ingredient is an enzyme.

The present invention according to claim 5 provides the granular composition according to claim 4, in which the enzyme is one or a combination of two or more selected from the group consisting of cellulase, amylase, protease, and lipase.

The present invention according to claim 6 provides the granular composition according to claim 5, in which cellulase is derived from Trichoderma viride.

The present invention according to claim 7 provides the granular composition according to claim 5, in which amylase is derived from Aspergillus oryzae.

The present invention according to claim 8 provides the granular composition according to claim 5, in which protease is derived from Aspergillus niger.

The present invention according to claim 9 provides the granular composition according to claim 5, in which lipase is derived from Candida cylindracea.

The present invention according to claim 10 provides the granular composition according to claim 1, in which the bioactive ingredient is an antibiotic.

The present invention according to claim 11 provides the granular composition according to claim 10, in which the antibiotic is colistin.

The present invention according to claim 12 provides the granular composition according to any one of claims 1 and 4 to 11, in which a content of the bioactive ingredient is 0.1 to 15% by weight.

The present invention according to claim 13 provides a method of producing a granular composition according to claim 1, comprising allowing a mixture containing a molten hardened oil and a bioactive ingredient to adhere to a granular sugar or to be formed into a film thereon.

The present invention according to claim 14 provides the method of producing a granular composition according to claim 13, in which the saccharide is granulated sugar or lactose.

The present invention according to claim 15 provides the method of producing a granular composition according to claim 13, in which the hardened oil is a hardened palm oil.

The present invention according to claim 16 provides the method of producing a granular composition according to claim 13, in which the bioactive ingredient is an enzyme.

The present invention according to claim 17 provides the method of producing a granular composition according to claim 16, in which the enzyme is one or a combination of two or more selected from the group consisting of cellulase, amylase, protease, and lipase.

The present invention according to claim 18 provides the method of producing a granular composition according to claim 13, in which the bioactive ingredient is an antibiotic.

The present invention according to claim 19 provides the method of producing a granular composition according to claim 18, in which the antibiotic is colistin.

The present invention according to claim 20 provides the method of producing a granular composition according to any one of claims 13 and 16 to 19, in which a content of the bioactive ingredient is 0.1 to 15% by weight.

The present invention according to claim 21 provides a pellet type feed obtained by compounding a granular composition of any one of claims 1 to 12.

The present invention according to claim 22 provides a method of producing a pellet type feed, comprising using a granular composition according to any one of claims 1 to 12.

Best Mode for carrying out the Invention

Hereinafter, the present invention will be described in detail.

The present invention according to claim 1 provides a granular composition including a core material and a layer that covers the core material, in which the core material is made of saccharides and the layer that covers the core material is made of a hardened oil and a bioactive ingredient.

The granular composition of the present invention is constructed such that a bioactive ingredient as an effective ingredient is not directly affected by wet heat by: suspending the bioactive ingredient in a hardened oil; and then allowing the mixture to adhere to an appropriate carrier or to be formed into a film thereon.

Consequently, the granular composition has wet-heat stability. The term "wet-heat stability" used herein means the property of the granular composition where, even in the case of subjecting a bioactive ingredient to the condition of humidification and/or heating, the activity of the bioactive ingredient contained in the composition is kept as it is by reducing the degree of reduction in activity of the bioactive ingredient under such conditions . More concretely, by taking an enzyme for example, the term refers to the fact that the enzyme keeps its activity 75% or more after being exposed one minute under a steam-heating condition at about 80°C. This heating condition is harder than other conditions, such as the process of steam sterilization of feed, which is usually conducted, and environmental conditions in a feed storage tank placed in the open air during summer.

Examples of the bioactive ingredient used in the present invention include compounds having effective biological activities in therapy or prevention of various diseases, such as enzymes, antibiotics, vaccines, hormones, and vitamins.

Preferable examples of the enzyme to which the present invention is applied include, but not limited to, cellulase, amylase, protease, and lipase. More preferable examples of the enzyme include cellulase derived from Trichoderma viride, amylase derived from Aspergillus oryzae, protease derived from Aspergillus niger, and lipase derived from Candida cylindracea. Those enzymes may be used independently or in combination of two or more thereof. Alternatively, two or more of the enzymes belonging to the same category may be used in combination.

The enzyme used in the present invention is generally in the form of powder and may be prepared from culture solutions obtained after incubating the respective enzyme-producing bacteria by the conventional methods. Alternatively, the enzyme may be a commercially available bulk powder thereof. For instance, with respect to cellulase, a preparation method thereof from a culture solution involves: incubating any of microorganisms capable of producing the enzyme, such as Aspergillus niger, Humicola insolens, Trichoderma viride, Acremonium cellulolyticus, Fusarium oxysporum, and Rhizopus oryzae; subjecting the resulting culture to, for example, centrifugation to obtain a supernatant fluid; if required, concentrating the supernatant fluid by means of ultrafiltration; and producing cellulase bulk powder of interest by a spray drying process or the like. In addition, other enzymes can be obtained similarly from culture solutions of the respective organisms capable of producing the enzymes, such as Aspergillus oryzae, Bacillus subtilis, and Bacillus licheniformis for amlylase, such as Aspergillus niger, Bacillus licheniformis, Bacillus subtilis, and Aspergillus oryzae for protease, and such as Candida cylindracea, Rhizopus japonicus, Rhizopus delemar, and Arthrobacter ureafaciens for lipase.

Next, examples of the antibiotic to which the present invention is applied include colistin, kanamycin, streptomycin, penicillin, and phosphomycin. In particular, of those, colistin is preferable. Those antibiotics may be used independently or in combination of two or more thereof.

Any of the antibiotics used in the present invention is generally in the form of powder and may be prepared from an organism capable of producing the antibiotic. For instance, colistin may be prepared from a culture solution obtained after incubating Bacillus polymyxa or may be commercially available antibiotic bulk powder.

Furthermore, examples of the hardened oil used in the present invention include: animal hardened oils, such as a beef tallow hardened oil and a lard hardened oil; and plant hardened oils such as a rapeseed hardened oil, a soybean hardened oil, an olive hardened oil, a hardened palm oil, and a castor bean hardened oil. Of those, the plant hardened oils are preferable. In particular, the hardened palm oil is preferable. By the way, drying oils, semi-drying oils, and non-drying oils are not preferable because these oils are difficult to handle due to their high viscosity and may penetrate core materials. On the other hand, the hardened oil can be molten at a temperature of about 50°C or more. Thus, a bioactive ingredient can be suspended in the hardened oil. In addition, the hardened oil can be easily solidified by lowering temperature to thereby cover a core material, while no penetration into the core material is observed.

Therefore, examples of an appropriate hardened oil used in the present invention include those having melting points of about 50°C or more, preferably about 50 to 65°C. In addition, the content of the hardened oil used in the present invention is generally in the range of 1 to 20% by weight, preferably in the range of 5 to 10% by weight.

In the present invention, ingredients that constitute a film layer for covering a core material may include an excipient and a binder if required in addition to a hardened oil and a bioactive ingredient.

Furthermore, saccharides are constituents of the core material of a granular composition. Examples of a saccharide used in the present invention include granulated sugar as a granular crystal of sucrose, lactose, yellow soft sugar, and sucrose. Of those, granulated sugar and lactose are preferable. In addition, they may be used independently or in combination of two or more thereof.

The content of a bioactive ingredient in a granular composition is generally in the range of 0.1 to 15% by weight, preferably in the range of 1 to 10% by weight. If the content of the bioactive ingredient is less than 0.1% by weight, the composition becomes inhomogeneous. If the content of the bioactive ingredient is more than 15% by weight, compaction may occur together with an increase in volume of a hardened oil.

Next, the method of producing a granular composition of the present invention, which is set forth in claim 13, will be described.

The production method of the present invention comprises allowing a mixture containing a molten hardened oil and a bioactive ingredient to adhere to a granular saccharide or to be formed into a film thereon.

The granular compositionhas comparatively simple composition and structure, so that the production step thereof is simple and has a small number of steps. Therefore, a decrease in activity of the pharmaceutical preparation-containing bioactive ingredient generally accompanied with an increase in number of steps can be remarkably reduced. In the present invention, the simple but most important process control is temperature control. That is, a temperature at the time of keeping a bioactive ingredient-mixed hardened oil prepared by mixing a bioactive ingredient provided as powder with a hardened oil after melting the hardened oil, a temperature of a saccharide at the time of allowing the bioactive ingredient-mixed hardened oil to adhere to the saccharide provided as a carrier or to be formed into a coating film thereon, a temperature of the bioactive ingredient composition after the adhesion to saccharide or the formation of the coating film thereon, and a temperature at the time of cooling to obtain a granular composition as a final product is specified. Consequently, a uniform structure of interest, which is stable against wet heat, can be obtained.

Concretely, in the production method of the present invention, a hardened oil is molten and then mixed with a bioactive ingredient to thereby obtain a bioactive ingredient-mixed hardened oil. On the other hand, the temperature of saccharide is kept at temperatures around the melting point of the hardened oil, then the bioactive ingredient-mixed hardened oil is gradually added, followed by cooling down to about 35°C. Thus, the bioactive ingredient-mixed hardenedoil is adhered to or formed into a film on the outerperipheral surface of the saccharide. At last, undesired large particles are removed through sifting or the like, and a desired granular composition can be obtained.

As far as the granular composition of the present invention has a configuration as a granular composition as described above, the production method thereof is not limited. In addition, machines, facilities, and so on used for the present invention are not particularly limited. Any of them suitable for the production method may be selected.

Next, the present invention set forth in claim 21 or 22 relates to a pellet type feed or a method of producing the same.

The production of a pellet type feed may be principally carried out by the conventional method. In the production process, the standard feed is mixed with the granular composition of the present invention to be sterilized and processed under wet-heat conditions The granular composition of the present invention has wet-heat stability as described above. Even if it is used for the production of pellet type feed to be subjected to a wet-heat process and steam sterilization, the bioactive ingredient is present stably and exerts the original capability against a domestic animal fed with the feed.

Practical examples and test examples of the granular compositions of the present invention will be exemplified below. However, the present invention is not limited to these examples.

Furthermore, the bioactive ingredients used in the examples and so on include cellulase bulk powder (manufactured by Meiji Seika Kaisha, Ltd.) derived from Trichoderma viride, amylase bulk powder (manufactured by Meiji Seika Kaisha, Ltd.) derived from Aspergillus oryzae, protease bulk powder (manufactured by Meiji Seika Kaisha, Ltd.) derived from Aspergillus niger, lipase bulk powder (manufactured by Meito Sangyo Co., Ltd.) derived from Candida cylindracea, colistin sulfate bulk powder (manufactured by Meiji Seika Kaisha, Ltd., LOT No. 55720, 709 μg (titer) /mg) derived from Bacillus polymyxa. In addition, the hardened oil used is hardened palm oil (extreme A) manufactured by New Japan Chemical Co., Ltd., the granulated sugar used is "Granulated Sugar G (particle size 0.25 to 1 mm) "manufactured by Nissin Sugar Manufacturing Co., Ltd., and lactose used is Lactose #100 manufactured by DMV International Co., Ltd.

Example 1 Preparation of granular composition A

Twenty grams of hardened palm oil was placed in a 100-mL glass beaker, which was in turn immersed in a thermostatic tank of 80°C ± 1°C, followed by stirring and dissolving the hardened palm oil with a spatula. Next, 6 g of cellulase bulk powder of Trichoderma viride was mixed with stirring, to prepare enzyme-mixed hardened oil.

On the other hand, 174 g of granulated sugar was placed in a 500-mL plastic beaker, which was maintained in a thermostatic tank at the temperature of 50°C ± 1°C, followed by gradually adding 26 g of the above enzyme-mixed hardened oil thereto. Subsequently, after the hardened oil was cooled to 35°C and solidified, undesired large particles were removed with a sieve having a mesh size of 1. 4 mm to give a desired granular composition A in which the content of cellulase of Trichoderma viride was 3% by weight.

Example 2 Preparation of granular composition B

Twenty grams of hardened palm oil was placed in a 100-mL glass beaker, which was in turn immersed in a thermostatic tank of 80°C ± 1°C, followed by stirring and dissolving the hardened palm oil with a spatula. Next, 6 g of cellulase bulk powder of Trichoderma viride was mixed with stirring, to prepare enzyme-mixed hardened oil.

On the other hand, 174 g of lactose was placed in a 500-mL plastic beaker, which was maintained in a thermostatic tank at the temperature of 50°C ± 1°C, followed by gradually adding 26 g of the above enzyme-mixed hardened oil thereto. Subsequently, after the hardened oil was cooled to 35°C and solidified, undesired large particles were removed with a sieve having a mesh size of 1.4 mm to give a desired granular composition B in which the content of cellulase of Trichoderma viride was 3% by weight.

Example 3 Preparation of granular composition C

After the temperature of 130.5 kg of granulated sugar was increased and adjusted to 55°C using a vibrating fluidized-bed dryer (VDF-6000; manufactured by Fuji Paudal), a belt feeder (manufactured by KYC Machine Industry) was used to add and mix the total amount thereof in the Mazelar mixer (PM-200VP; manufactured by Mazelar), the temperature of which was maintained with an electrothermal heater.

On the other hand, the total amount of 15 kg of hardened palm oil was charged into a hardened oil-dissolving tank (T.K. UNI-MIXER; manufactured by TOKUSHU KIKA KOGYO) and dissolved while the liquid temperature was regulated at 80°C ± 3°C. In the tank, 4.5 kg of cellulase bulk powder of Trichoderma viride was then added and mixed to prepare enzyme-mixed hardened oil.

Then, after the temperature of the granulated sugar in the Mazelar mixer was stabilized at 55°C ± 1°C, the enzyme-mixed hardened oil was fed into an injection nozzle which was kept warm while the granulated sugar was mixed. The total amount (19.5kg) of the mixture was poured over approximately 15 minutes. Following that, the contents were mixed for about 10 minutes for even dispersion. At this time, the temperature of the contents reached approximately 60°C.

Next, while cold air was fed with the use of a spot air conditioner (manufactured by Hitachi Air Conditioning System) to mix the contents, the temperature thereof was cooled to 56°C ± 1°C. Then, the mixture was quickly transferred from the Mazelar mixer to a concrete mixer (PT-200; manufactured by Mazelar) and cooled using the spot air conditioner while being mixed until the temperature reached 35°C or lower.

Subsequently, undesired large particles were removed by sieving the mixture with a round sieve (cartridge type; manufactured by DALTON) to give a desired granular composition C in which the content of cellulase of Trichoderma viride was 3% by weight.

Example 4 Preparation of granular composition D

After the temperature of 123 kg of granulated sugar was adjusted to 57°C, a belt feeder was used to add and mix the total amount thereof in the Mazelar mixer kept warm with an electrothermal heater. The temperature of the contents at this step was controlled at 55°C.

On the other hand, the total amount of 18 kg of hardened palm oil was charged into a hardened oil-dissolving tank and dissolved while the liquid temperature was regulated at 81°C ± 2°C. In the tank, 9 kg of cellulase bulk powder of Trichoderma viride was then added and mixed to prepare enzyme-mixed hardened oil.

Following the above steps, after the temperature of the granulated sugar in the Mazelar mixer was stabilized at 57°C ± 1°C, the enzyme-mixed hardened oil was fed into the injection nozzle which was kept warm while the granulated sugar was mixed. The total amount (27 kg) of the mixture was poured over approximately 20 minutes. Thereafter, the contents were mixed for approximately 10 minutes for even dispersion. At this time, the temperature of the contents reached approximately 58°C.

Next, while cold air was fed with the use of a spot air conditioner to mix the contents, the temperature thereof was cooled to 56°C ± 1°C. Then, the mixture containing the enzyme was quickly transferred from the Mazelar mixer to a concrete mixer and carefully mixed so that there were not accumulations left. The temperature of the mixture was cooled to 35 °C or lower with the spot air conditioner. Subsequently, undesired large particles were removed with the DALTON sieving machine to give a desired granular composition D in which the content of cellulase of Trichoderma viride was 6% by weight.

Example 5 Preparation of granular composition E

Twenty grams of hardened palm oil was placed in a 100-mL glass beaker, which was in turn immersed in a thermostatic tank of 80°C ± 1°C, followed by stirring and dissolving the hardened palm oil with a spatula. Next, 6 g of amylase bulk powder of Aspergillus oryzae was mixed with stirring, to prepare enzyme-mixed hardened oil.

On the other hand, 174 g of granulated sugar was placed in a 500-mL plastic beaker, which was maintained in a thermostatic tank at the temperature of 50°C ± 1°C, followed by gradually adding 26 g of the above enzyme-mixed hardened oil thereto. Subsequently, after the hardened oil was cooled to 35°C and solidified, undesired large particles were removed with a sieve having a mesh size of 1.4 mm to give a desired granular composition E in which the content of amylase of Aspergillus oryzae was 3% by weight.

Example 6 Preparation of granular composition F

Twenty grams of hardened palm oil was placed in a 100-mL glass beaker, which was in turn immersed in a thermostatic tank of 80°C ± 1°C, followed by stirring and dissolving the hardened palm oil with a spatula. Next, 6 g of protease bulk powder of Aspergillus niger was mixed with stirring, to prepare enzyme-mixed hardened oil.

On the other hand, 174 g of granulated sugar was placed in a 500-mL plastic beaker, which was maintained in a thermostatic tank at the temperature of 50°C ± 1°C, followed by gradually adding 26 g of the above enzyme-mixed hardened oil thereto. Subsequently, after the hardened oil was cooled to 35°C and solidified, undesired large particles were removed with a sieve having a mesh size of 1.4 mm to give a desired granular composition F in which the content of protease of Aspergillus niger was 3% by weight.

Example 7 Preparation of granular composition G

Fifty grams of hardened palm oil was placed in a 100-mL glass beaker, which was in turn immersed in a thermostatic tank of 80°C ± 1°C, followed by stirring and dissolving the hardened palm oil with Three-One Motor (600G type; HEIDON) . Next, 15 g of lipase bulk powder of Candida cylindracea was mixed with stirring to prepare enzyme-mixed hardened oil.

On the other hand, 435 g of granulated sugar was placed in a high-speed mixer (LFS-GS-2J type; FUKAE POWTEC), which was maintained at 55°C ± 1°C in a hot-water reflux jacket, followed by gradually adding 65 g of the above enzyme-mixed hardened oil thereto. Subsequently, after the hardened oil was cooled to 35°C and solidified, undesired large particles were removed with a sieve having a mesh size of 1.4 mm to give a desired granular composition G in which the content of lipase of Candida cylindracea was 3% by weight.

Example 8 Preparation of granular composition H

Thirty grams of hardened palm oil was placed in a 100-mL glass beaker, which was in turn immersed in a thermostatic tank of 80°C ± 1°C, followed by stirring and dissolving the hardened palm oil with Three-One Motor (600G type; HEIDON) . Next, 8.5 g of colistin sulfate bulk powder was mixed with stirring to prepare colistin sulfate-mixed hardened oil.

On the other hand, 261.5 g of granulated sugar, which was kept warm in a hot-air drier of 60°C, was transferred to a high-speed mixer (LFS-GS-2J type; FUKAE POWTEC) and maintained at 50 to 60°C in a hot-water reflux jacket, followed by gradually adding 38.5 g of the colistin sulfate-mi xedhardened oil thereto. Subsequently, after the hardened oil was cooled to 35°C and solidified, undesired large particles were removed with a sieve having a mesh size of 1 .4 mm to give a desired granular composition H in which the content of colistin sulfate was 2.8% by weight.

Comparative Example 1 Preparation of powdery composition a

Using a high-speed stirring mixer (SEGV-200), 471.8 g of oil cake of rice bran, 28.2 g of colistin sulfate bulk powder, and 500 g of oil cake of rice bran were successively added in this order and rotated at 30 rpm for 10 minutes to give a desired powdery composition a in which the content of colistin sulfate was 2.8% by weight.

Test Example 1

In this example, granular compositions of a variety of enzymes were prepared to evaluate the compositions for the stability of the enzymes.

[Method of measuring enzyme activity and method of preparing sample]

1-1. Measurement of cellulase activity

Cellulase was extracted with an acetic acid buffer (pH 4.5) and used as a sample solution which was in turn reacted with CM-CELLULOSE (manufactured by Megazyme) coupled with blue dye. This colored CM-CELLULOSE can be used in a powder form. The colored CM-CELLULOSE was treated with an enzyme under the condition of pH 4.5 at 40°C for 10 minutes and the reaction was then terminated with acidic alcohol.

Then, the amount of the blue dye in a supernatant obtained from the reaction solution by centrifugation was measured in terms of absorbance (620 nm) to determine a value derived by dividing the absorbance by absorbance obtained from a blank solution. Similarly, cellulase activity (unit: u/mL) in the sample was determined on the basis of the calibration curve of a cellulase standard solution reacted with the enzyme.

1-2. Measuring range

The sample solution was diluted to thereby adjust cellulose glycosylation activity to 0.05 to 0.3 u/mL. The measuring range of this cellulose glycosylation activity corresponds to 10 to 10, 000 u/g as the enzyme contained in the enzyme composition.

1-3. Method of preparing sample solution

Approximately 1 g of a cellulase sample was placed in a 100-mL Erlenmeyer flask. The flask was supplemented with 50 mL of an acetic acid buffer (pH 4.5) and then sealed. The mixture was stirred at room temperature for 60 minutes with a magnetic stirrer and filtered with a 0.45-μm membrane filter (Maillex-HV, pore size: 25 mm; manufactured by MILLIPORE). The filtered mixture was used as a sample solution.

1-4. Cellulase standard solution

The solution was prepared from the cellulase of Trichoderma viride having known cellulose glycosylation activity with a use of an acetic acid buffer (pH 4.5) so that its activity could exactly be indicated in the range of 0.05 to 0.3 u/mL.

2-1. Measurement of amylase activity

Amylase activity (starch glycosylation power) was tested in conformance with the "test method on enzyme power" as a general test method of Ministerial Ordinance on standards of ingredients of feed and feed Additives (Ministry of Agriculture and Forestry's Ordinance No. 35 of 1976). However, the measurement was carried out at pH 5.0. A sample solution was prepared with 0.1 mol/L lactate buffer. One unit of starch glycosylation power corresponds to the amount of an enzyme that effects increase in reducing power corresponding to 1 mg of glucose for 1 minute in the initial stage of the reaction when amylase acts on potato starch at 37°C.

2-2. Measuring range

The sample solution is diluted and thereby prepared so that the concentration per mL is brought to 0.4 to 0.8 units of starch glycosylation power.

2-3. Preparation of substrate solution

Approximately 1 g of potato starch was precisely weighed in advance and dried at 105°C for 2 hours to measure its weight loss. Potato starch corresponding to 1 g of the dried product was exactly weighed and placed in an Erlenmeyer flask. To the flask, 20 mL of water was added and 5 mL of 2 mol/L sodium hydroxide test solution was gradually added, with the flask sufficiently shaken, to form the mixture into paste. Subsequently, after heating for 3 minutes withstirringinawaterbath, 25mL of water was added. After cooling, the mixture was accurately neutralized with 2 mol/L hydrochloric acid test solution and supplemented with 10 mL of 0.1 mol/L acetic acid/sodium acetate buffer adjusted to pH 5.0 and subsequently with water to bring the mixture to exactly 100 mL.

2-4. Procedures

An appropriate amount of the sample is precisely weighed and dissolved by the addition of 0.1 mol/L lactate buffer adjusted to pH 5.0 so that the concentration per mL is brought to 0.4 to 0.8 units of starch glycosylation power. The resulting mixture is used as a sample solution. Filtration or centrifugation is carried out, if necessary. Then, 10 mL of the substrate solution is exactly weighed and placed in a test tube having a diameter of 30 mm. After being left at $37 \pm 0.5$°C for exactly 10 minutes, the test tube is exactly supplemented with 1 mL of the sample solution, immediately shaken to mix the solution, and left at $37 \pm 0.5$°C for exactly 10 minutes.
Next, 2 mL of an alkaline tartrate solution of Fehling's solution is added and the whole is immediately stirred. After additional 2 mL of a copper solution of Fehling's solution is exactly added and the whole is mixed by lightly shaking, a funnel is placed on the mouth of the test tube and the test tube is heated in a water bath for exactly 15 minutes. Immediately after that, the test tube is cooled to 25°C or lower with running water. Further, 2 mL of a concentrated potassium iodide test solution and 2 mL of diluted 3 mol/L sulfuric acid are added, and liberated iodine is titrated with 0. 05 mol/L sodium thiosulfate solution (indicator: 1 to 2 drops of a soluble starch test solution) . In this case, the point where blue color in the solution has disappeared is designated as the end point of the titration and its titer is designated as A mL. Alternatively, 10 mL of water is taken instead of 10mL of the substrate solution and then manipulated in the same way as above. Its titer is designated as B mL. According to the formula below, the unit of starch glycosylation power (U) in 1 g is determined. Here, w in the formula is the amount of the sample (g) in 1 mL of the sample solution.

$$U = (B-A) \times 1.6 \times 1/10 \times 1/w$$

3-1. Measurement of protease activity

Protease activity (power to digest proteins) was tested in conformance with the "test method on enzyme power (test method on power to digest proteins, the second method)" as the general test method of Ministerial Ordinance on standards of ingredients of feed and feed Additives (Ministry of Agriculture and Forestry' s Ordinance No. 35 of 1976). However, the measurement was carried out at pH 2.6. A sample solution was prepared with 0.1 mol/L acetate buffer. One unit of power to digest proteins corresponds to the amount of an enzyme that effects increase in the non-protein color substance of Folin's reagent corresponding to 1 μg of tyrosine for 1 minute in the initial stage of the reaction when protease acts on whey casein at 37°C.

3-2. Measuring range

The sample solution is diluted and thereby prepared so that the concentration per mL is brought to 10 to 30 units of power to digest proteins.

3-3. Method of preparing substrate solution

Approximately 1 g of whey casein is precisely weighed in advance and dried at 105°C for 2 hours to measure its weight loss. Whey/casein corresponding to 1.20 g of the dried product is exactly weighed and supplemented with 16 mL of 1 mol/L lactic acid test solution and 146 mL of water. The mixture is heated in a water bath to be dissolved. After being cooled with running water, this solution is supplemented with 1 mol/L hydrochloric acid test solution or 1 mol/L sodium hydroxide test solution and adjusted to pH2.6, followed by the addition of water to bring the solution to exactly 200 mL.

3-4. Production of calibration curve

A tyrosine standard product is dried at 105°C for 3 hours, and 0.500 g thereof is exactly weighed and dissolved by adding 2 mol/L hydrochloric acid test solution to bring the solution to exactly 500 mL. This solution is exactly weighed at 1 mL, 2 mL, 3 mL, and 4 mL, each of which is supplemented with 0.2 mol/L hydrochloric acid test solution to bring the solution to exactly 100 mL.
Subsequently, 2 mL of each of the solutions is exactly weighed and supplemented with 5 mL of 0.55 mol/L sodium carbonate test solution and 1 mL of Folin' s reagent diluted three fold with water, respectively. After the solutions are left at $37 \pm 0.5$°C for 30 minutes, the absorbance of each of those solutions is measured at a wavelength of 660 nm to measure the amounts ($A_1$, $A_2$, $A_3$, and $A_4$) of the color substances of the Folin's reagent in the solutions, respectively. Alternatively, 2 mL of 0.2 mol/L hydrochloric acid test solution is exactly weighed and then manipulated in the same way as above to measure absorbance $A_0$.
A calibration curve is produced with the differences between the absorbances ($A_1-A_0$), ($A_2-A_0$), ($A_3-A_0$), and ($A_4-A_0$) as ordinate against the amount (μg) of tyrosine as abscissa.

3-5. Procedures

An appropriate amount of the sample is precisely weighed and dissolved by the addition of water, 0.1 mol/L lactate buffer, acetic buffer, or 0.01 mol/L acetic acid/sodium acetate buffer so that the concentration per mL is brought to 10 to 30 units of power to digest proteins . The resultingmixture is used as a sample solution. Filtration or centrifugation is carried out, if necessary. Then, 5 mL of the substrate solution is exactly weighed and left at 37°C $\pm$ 0.5°C for 30 minutes. In addition, after this solution is filtered to completely remove precipitates, 2 mL of the filtrate is exactly weighed and supplemented with 5 mL of 0.55 mol/L sodium carbonate test solution and 1 mL of Folin' s reagent diluted three fold with diluted water. The resulting solution is sufficiently stirred and left at $37 \pm 0.5$°C for 30 minutes, and then the absorbance AT of this solution is measured at a wavelength of 660 nm. Separately, 1 mL of the sample solution is exactly weighed and supplemented with 5 mL of the trichloroacetic acid test solution A. After being sufficiently shaken to mix the solution, the solution is exactly supplemented with 5 mL of the substrate solution and left at $37 \pm 0.5$°C for 30 minutes. Then, the solution is manipulated in the same way as above to measure the absorbance $A_T$. According to the formula below, the unit of power to digest proteins (U) in 1 g is determined. Here, F in the formula is the amount of tyrosine (μg) for the absorbance difference=1 determined by the calibration curve, and w is the amount of the sample (g) in 1 mL of the sample solution.

$$U = (A_T - A_{T'}) \times F \times 11/2 \times 1/10 \times 1/w$$

4-1. Measurement of lipase activity

Lipase activity (power to digest fats) was tested in conformance with the "test method on enzyme power" as the general test method of Ministerial Ordinance on standards of ingredients of feed and feed Additives (Ministry of Agriculture and Forestry's Ordinance No. 35 of 1976). However, the measurement was carried out at pH 7.0. One unit of power to digest fats corresponds to the amount of an enzyme that effects increase in power to digest corresponding to 1 μmol of fatty acid for 1 minute in the initial stage of the reaction when lipase acts on olive oil at 37°C.

4-2. Measuring range

The sample solution is diluted and thereby prepared so that the concentration per mL is brought to 1.0 to 5.0 units of power to digest fats.

4-3. Method of preparing substrate solution

In the 500-mL container of an emulsifier, 200 to 300 mL of a polyvinyl alcohol test solution/olive oil (3:1) mixture solution is placed and emulsified for 10 minutes by cooling to 10°C while being rotated at 12,000 to 16,000 rpm. The resulting solution is left in the dark for 1 hour and used after it has been confirmed that oil layers are not separated.

4-4. Procedures

An appropriate amount of the sample is precisely weighed and dissolved by the addition of cold water so that the concentration per mL is brought to 1.0 to 5.0 units of power to digest fats. The resulting mixture is used as a sample solution. Filtration or centrifugation is carried out, if necessary. After 5 mL of the substrate solution and 4 mL of 0.1 mol/L phosphate buffer adjusted to pH 7.0 are exactly weighed and sufficiently mixed, the mixture is left at 37°C ± 0.5°C for 10 minutes and then supplemented with 1 mL of the sample solution. Immediately after that, the mixture is stirred and left at 37°C ± 0.5°C for exactly 20 minutes.
Next, 10 mL of an acetone/ethanol (1:1) mixture solution is added and the whole is mixed by shaking. The solution is titrated with 0.05 mol/L hydrochloric acid (indicator: a few drops of a phenolphthalein test solution). In this case, the point where red color in the solution has disappeared is designated as the end point of the titration and its titer is designated as A mL. Separately, 5 mL of the substrate solution and 4 mL of 0.1 mol/L phosphate buffer adjusted to pH 7.0 are exactly weighed and sufficiently mixed, and then are left at 37°C ± 0.5°C for 30 minutes.
Next, 10 mL of an acetone/ethanol (1:1) mixture solution is exactly added and the whole is stirred. Further, 1 mL of the sample solution is exactly added and shaken to mix the solution. The resulting solution is then manipulated in the same way as above and its titer is designated as B mL. According to the formula below, the unit of power to digest fats (U) in 1 g is determined. Here, w in the formula is the amount of the sample (g) in 1 mL of the sample solution.

$$U = 50 \times (B-A) \times 1/20 \times 1/w$$

[Evaluation of granular composition for stability of enzyme]

The granular compositions prepared above were evaluated for the stability of enzymes by a method described below.
The step of wet heat treatment carried out in the step of forming pellet that was adopted for producing feed for animals (livestock and fishes) was reproduced in a laboratory as described below to evaluate the enzyme compositions for the stability of enzymes. That is, approximately 0.5 g of each of the samples was placed in a glass vessel and supplemented and mixed with 1 g of oil cake of rice bran previously allowed to contain water. The resulting mixture was then placed on the shelf of a dry heater (warm air-circulating type) of 100°C and subjected to wet heat treatment for 8 minutes, followed by determining the survival rate of enzyme activity for the stability of enzyme of each enzyme composition according to each test method on enzyme power described above.

Moreover, for comparison, those obtained from each enzyme bulk powder of cellulase, amylase, protease, and lipase that was mixed with oil cake of rice bran and then subjected to wet heat treatment as described above were used as powdery compositions to determine the survival rate of enzyme activity in the same way. The result is shown in Table 1.

Table 1

| Enzyme name | survival rate of enzyme activity(%) | | |
|---|---|---|---|
| | Example No. | Granular composition | Powdery composition |
| Cellulase | Example 1 | 84.5 | 10.3 |
| Amylase | Example 5 | 63.4 | 0.0 |
| Protease | Example 6 | 43.8 | 0.0 |
| Lipase | Example 7 | 51.0 | 0.6 |

This result shows that the granular compositions of cellulase, amylase, protease, and lipase according to the present invention have high survival rates of enzyme activity after wet heat treatment.

Test Example 2

In this example, the stability of enzymes was compared between a commercially available enzyme standard product and the granular composition according to the present invention. That is, the stability of enzymes was compared between commercially available cellulase (RONOZYME VP, manufactured by Roche, Lot No. KT902015) and the granular composition according to the present invention using the analytical method described in Test Example 1. The result is shown in Table 2.

Table 2

| | Sample Name | Cellulase residual ratio (%) |
|---|---|---|
| Example 3 | Granular composition C | 76 |
| Commercially available cellulase | RONOZYME | 7 |

The result in Table 2 shows that the granular composition of the present invention has significantly better stability than that of the commercially available enzyme standard product as a control to be compared.

Test Example 3

In this example, a pellet type feed was prepared by compounding the granular composition of the present invention and the wet-heat stability of the granular composition in the pellet type feed was then investigated.

To 100 kg of Piggy Standard Feed (manufacturedbyNippon Formula Feed Manufacturing Co. Ltd.), 1.4% by weight or 2.8% by weight of the granular composition C prepared in Example 3 was added using a pellet-feed manufacturing machine (1100 series, manufactured by California Pellet Mill Co., Ltd.) at steam temperatures of 74 to 86°C to prepare a pellet type feed.

Feedwas recovered after the pellet type feed had been confirmed to reach a temperature of 78 to 80°C. After the collection of the feed, in consideration of the wet-heat hysteresis thereof, the feed was immediately cooled with cold air from a spot air conditioner to thereby provide an assay sample.

As a control, pellet type feed was produced by the same way as described above, except that commercial cellulase (RONOZYME VP, manufactured by Roche Co., Ltd. Lot No. KT902015) was used. Then, the comparison of wet-heat stability was carried out using the method described in Test Example 1 on the basis of cellulase activity residual ratios. The results are shown in Table 3.

Table 3

| Sample Name | Cellulase activity residual ratio (%) |
|---|---|
| Pellet type feed containing 1.4% by weight of granular composition C | 12 |

Table 3   (continued)

| Sample Name | Cellulase activity residual ratio (%) |
|---|---|
| Pellet type feed containing 2.8% by weight of granular composition C | 23 |
| Pellet type feed containing 1. 4% by weight of commercial cellulase | 2 |
| Pellet type feed containing 2.8% by weight of commercial cellulase | 11 |

The results of Table 3 show that the granular composition of the present invention is more stable than a commercial product in producing a pellet type feed.

Test Example 4

In this example, the stability of granular compositions prepared using antibiotics was evaluated.

[Titer measuring method (High performance liquid chromatographic method)

1. Sample preparation method

In each of 200 mL volume screws cap Erlenmeyer flasks, about 1 g of a sample was collected precisely. Each of the flasks was correctly added with 2 mL of an internal standard solution and then added with 98 mL of a 10 g/dl phosphate buffer (pH 6.0), followed by being sealed hermetically. Subsequently, the flask was shaken for 30 minutes and then left standing to obtain a supernatant. The supernatant was filtrated through a 0.45 μm membrane filter (25 mm in pore size, Maillex-HV, manufactured by MILLIPORE Co., Ltd.) to thereby provide a filtrate as a sample solution.
Approximately 20 mg (titer) of colistin sulfate general-purposestandard product was precisely weighed and dissolved in about 50 mL of 10 g/dL phosphate buffer (pH 6.0) . Then, 2 mL of the internal standard solution was correctly added. After that, 10 g/dL phosphate buffer (pH 6. 0) was added to obtain a total amount of 100 mL. The resultant was filtrated through a 0.45 μm membrane filter, and the filtrate was used as a standard solution.

2. Analytical method

Each 10 μl of the sample solution and the standard solution was tested on the condition described below by liquid chromatography. The ratios $Q_T$ and $Q_s$ of the total peak area of the colistin sulfates A and B to the peak area of the internal standard substance in each of the solutions were determined to calculate the amount of the colistin sulfate in the sample solution [μg (titer)/mL] according to the following formula.

The amount of the colistin sulfate in the sample solution [μg

(titer)/mL] = the amount of the colistin sulfate in the standard

solution [μg (titer) /mL] X $Q_T/Q_S$.

(Procedure condition)

Instrument used: general-purpose liquid chromatograph and automatic integrator.
Detector: ultraviolet absorption photometer (measurement wavelength: 215 nm).
Column: a column (Inertsil ODS-3; manufactured by GL Sciences) in which 5 μm of octadecylsilylated silica gel for liquid chromatograph was fed into a stainless tube having an internal diameter of 4.6 mm and a length of approximately 250 mm was used.
Temperature of column: a constant temperature around 25°C.
Mobile phase: 0.05 mol/L sodium sulfate solution and acetonitrile for HPLC were mixed in the ratio of 76:24 and adjusted to pH 2.3 with 1 mol/L sulfuric acid.
Flow rate: the retention time of the colistin sulfate B was set to 7 to 8 minutes.

The evaluation of the granular composition for stability was practiced essentially in the same way as in the above-described Test Example 1, except that: each 1 g of the granular composition H (Example 8) of the present invention and the powdery composition a of Comparative Example 1 used as samples was employed; and wet heat treatment was carried out by placing the sample on the shelf of a dry heater (warm air-circulating type) of 100°C for 60 minutes. The result is shown in Table 4.

Table 4

| Sample Name | Survival rate of colistin titer (%) |
|---|---|
| Granular composition H (Example 8) | 81.5 |
| Powdery composition a (Comparative Example 1) | 65.5 |

The result shows that the granular composition of colistin according to the present invention has a higher survival rate of colistin titer after wet heat treatment than that of the powdery composition as a control.

Industrial Applicability

The granular composition of the present invention has bioactive components such as an enzyme and an antibiotic as active ingredients, and this composition has excellent stability under the conditions of high temperature and high humidity. Therefore, the granular composition is highly practical in fields such as medicals, foods, and detergents, and is further expected to be utilized for the production of pellet type feed, and so on, in the field of feed.
Moreover, according to the production method of the present invention, the above-described granular composition can efficiently be produced in convenient steps.

**Claims**

1. A granular composition comprising a core material and a layer that covers the core material, wherein the core material is made of saccharides and the layer that covers the core material is made of a hardened oil and a bioactive ingredient.

2. The granular composition according to claim 1, wherein the saccharide comprises granulated sugar or lactose.

3. The granular composition according to claim 1, wherein the hardened oil comprises a hardened palm oil.

4. The granular composition according to claim 1, wherein the bioactive ingredient comprises an enzyme.

5. The granular composition according to claim 4, wherein the enzyme comprises one or a combination of two or more selected from the group consisting of cellulase, amylase, protease, and lipase.

6. The granular composition according to claim 5, wherein cellulase is derived from Trichoderma viride.

7. The granular composition according to claim 5, wherein amylase is derived from Aspergillus oryzae.

8. The granular composition according to claim 5, wherein protease is derived from Aspergillus niger.

9. The granular composition according to claim 5, wherein lipase is derived from Candida cylindracea.

10. The granular composition according to claim 1, wherein the bioactive ingredient comprises an antibiotic.

11. The granular composition according to claim 10, wherein the antibiotic comprises colistin.

12. The granular composition according to any one of claims 1 and 4 to 11, wherein a content of the bioactive ingredient is 0.1 to 15% by weight.

13. A method of producing a granular composition according to claim 1, comprising allowing a mixture containing a molten hardened oil and a bioactive ingredient to adhere to a granular saccharide or to be formed into a film thereon.

**14.** The method of producing a granular composition according to claim 13, wherein the saccharide comprises granulated sugar or lactose.

**15.** The method of producing a granular composition according to claim 13, wherein the hardened oil comprises a hardened palm oil.

**16.** The method of producing a granular composition according to claim 13, wherein the bioactive ingredient comprises an enzyme.

**17.** The method of producing a granular composition according to Claim 16, wherein the enzyme comprises one or a combination of two or more selected from the group consisting of cellulase, amylase, protease, and lipase.

**18.** The method of producing a granular composition according to claim 13, wherein the bioactive ingredient comprises an antibiotic.

**19.** The method of producing a granular composition according to claim 18, wherein the antibiotic comprises colistin.

**20.** The method of producing a granular composition according to any one of claims 13 and 16 to 19, wherein a content of the bioactive ingredient is 0.1 to 15% by weight.

**21.** A pellet type feed obtained by compounding a granular composition according to any one of claims 1 to 12.

**22.** A method of producing a pellet type feed, comprising using a granular composition according to any one of claims 1 to 12.

<div style="text-align:center">**INTERNATIONAL SEARCH REPORT**</div>

| International application No. |
| --- |
| PCT/JP03/14895 |

**A. CLASSIFICATION OF SUBJECT MATTER**
Int.Cl⁷ A23K1/16, A23K1/17, C12N9/42, C12N9/26, C12N9/50,
C12N9/20, C12N9/96, C12N9/98

According to International Patent Classification (IPC) or to both national classification and IPC

**B. FIELDS SEARCHED**

Minimum documentation searched (classification system followed by classification symbols)
Int.Cl⁷ A23K1/00-3/04, C12N9/00-9/99, C11D1/00-19/00

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 57-65171 A (Asama Chemical Co., Ltd.), 20 April, 1982 (20.04.82), (Family: none) | 1-22 |
| Y | JP 62-257990 A (Kao Corp.), 10 November, 1987 (10.11.87), (Family: none) | 1-22 |
| Y | JP 60-37983 A (Showa Denko Kabushiki Kaisha), 27 February, 1985 (27.02.85), (Family: none) | 1-22 |
| Y | JP 4-234985 A (Shin Nihon Kagaku Kogyo Kabushiki Kaisha), 24 August, 1992 (24.08.92), (Family: none) | 5-9,17 |

☒ Further documents are listed in the continuation of Box C. ☐ See patent family annex.

| | |
| --- | --- |
| * Special categories of cited documents: <br> "A" document defining the general state of the art which is not considered to be of particular relevance <br> "E" earlier document but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br> 09 January, 2004 (09.01.04) | Date of mailing of the international search report <br> 27 January, 2004 (27.01.04) |
| Name and mailing address of the ISA/ <br> Japanese Patent Office | Authorized officer |
| Facsimile No. | Telephone No. |

Form PCT/ISA/210 (second sheet) (July 1998)

**INTERNATIONAL SEARCH REPORT**

| International application No. |
|---|
| PCT/JP03/14895 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2-164044 A  (Nippon Soda Co., Ltd.),<br>22 June, 1990 (22.06.90),<br>(Family: none) | 21,22 |

Form PCT/ISA/210 (continuation of second sheet) (July 1998)